# EUROPEAN PATENT SPECIFICATION

(11) **EP 2 040 684 B1**
(45) Date of publication and mention of the grant of the patent: **23.01.2013**
(21) Application number: 07765142.0
(22) Date of filing: 09.07.2007
(51) Int. Cl.: A61K 9/20

(54) **MULTIPLE UNIT TABLETS**
TABLETTEN MIT MEHREREN EINHEITEN
TABLETTE AUX UNITES MULTIPLES

(30) Priority: 11.07.2006 EP 06116993
(43) Date of publication of application: 01.04.2009
(73) Proprietor: LEK Pharmaceuticals d.d., 1526 Ljubljana (SI)
(72) Inventor: STANIC LJUBIN, Tijana, 1000 Ljubljana (SI); KOCEVAR, Klemen, 1360 Vrhnika (SI)
(74) Representative: Oser, Andreas
(86) International application number: PCT/EP2007/006066
(87) International publication number: WO 2008/006534

(56) References cited:
- EP-A2- 0 106 443
- WO-A-01/34684
- WO-A-96/22082
- FR-A1- 2 785 538
- US-A1- 2005 191 325

## Description

The present invention belongs to the field of pharmaceutical technology and relates to multiple unit tablets comprising multiple units compacted together with at least two tablet filler-binders and other pharmaceutically acceptable excipients, wherein the particle size of tablet filler-binders differs from each other and is smaller than the size of multiple units.
The present invention further relates to a process for the preparation of multiple unit tablets.

Multiple unit dosage forms are multiparticulate drug delivery systems consisting of plurality of pellets, granules, spherules, micro spheres, mini tablets and other drug substance containing agglomerations or particles that can be loaded into either a capsule or tablet. Single unit dosage forms are traditional tablets and powder-filled capsules. Multiple unit dosage forms offer numerous significant therapeutic advantages over traditional single unit dosage forms, that is known from the literature, such as Pharmaceutical Pelletization Technology (Ghebre-Sellassie, I., ed.), Marcel Dekker Inc., New York, 1989, pages 1-11, Encyclopedia of Pharmaceutical Technology (Swarbrick, J., Boylan, J.C., ed.), Marcel Dekker Inc., New York, 1988, Vol. 11, pages 369-393, International Journal of Pharmaceutcs, volume 147 (1997), pages 95-107 and International Journal of Pharmaceutics, volume 114 (1995), pages 1-11. Multiple units generally disperse freely in the gastrointestinal tract and behave like liquids, leaving the stomach within a short period of time, which results in:
- improved biopharmaceutical characteristics, such as improved bioavailability, reduced the effect of the food on plasma profiles and in such way reduced intra- and inter-subject variability of plasma profiles,
- reduced peak plasma fluctuations and in such way minimized potential side effects,
- minimized local irritation in gastrointestinal tract.
When formulated as modified release dosage forms, multiple units perform improved safety and efficacy, since they are less susceptible to dose dumping than single unit formulations with modified release. The kinetics of the drug release from multiple unit dosage form is more stable than from the single unit dosage form, because the kinetics of the drug release from multiple unit dosage form is the average value of the kinetics of the drug release from individual subunits.

Multiple unit dosage forms are commonly filled into hard capsules. The alternative method of formulating multiple units is compacting into multiple unit tablets. This approach becomes increasingly important in pharmaceutical industry because of combining the advantages of multiple unit dosage forms and tablets in one final dosage form. The advantages of tablets over hard capsules are numerous:
- The production costs for capsules are high compared with those of tablets, due to higher cost of capsule shelves lower production rate of capsule-filling machines;
- Higher dose strengths can be administered to patients, since large volume tablets generally have greater patient acceptability than large volume capsules, which ensures better patient compliance;
- Capsules can not be divided in the same way as tablets;
- The use of already existing tableting capacities is also one of the reasons for preferring the development of a tablet instead of a capsule.

Two main challenges of formulating of multiple units into multiple unit tablets are:
- Tableting of multiple units into multiple unit tablets is mechanically stressful process for multiple units that are likely to be mechanically damaged during compression step of tableting. The damage of the multiple units during compression step of tableting can result in altered drug release. This is especially critical when multiple units coated with functional coating that assures modified release are tableted into multiple unit tablets, because compression step of tableting often results in rupture of functional coating.
- During the compression step of tableting of pellets with tableting excipents the particle segregation tends to occur, because of different size and shape of multiple units and particles of excipients. The particle segregation results in inappropriate content and mass uniformity of multiple unit tablets.

Therefore, two main requirements that must be met when formulating multiple unit tablets are that drug release from multiple unit tablets is not altered compared to the drug release from multiple units prior to tableting, and that the content and mass uniformity of multiple unit tablets complies with pharmacopoeia prescriptions.
Additionally, multiple unit tablets must be coherent and have appropriate hardness and friability, so that they could be further handled, such as coated and packed.

There are two approaches in multiple units tableting:
- tableting of multiple units without other excipients for tableting, and
- tableting of multiple units together with pharmaceutically exeptable excipients.

The approach of compacting of only multiple units without other excipients for tableting does not include the problem of particle segregation, but includes very difficult formulation of multiple units and the coating of multiple units. Multiple unit cores must be deformable enough so that they form coherent tablets, and the coatings of multiple units must be able to withstand compacting without damages, which can be ensured by formulating the coating of multiple units in such way that the coating posses improved elasticity.

The approach of compacting of multiple units together with pharmaceutically acceptable tableting excipients moderates requirements for the multiple units coating elastic properties, since plastically deformable or soft tableting excipients are able to partly absorb compaction forces and protect multiple units from mechanical damages. This approach enables also easier obtaining of coherent multiple unit tablets that have appropriate hardness and friability, because tableting excipients have larger surface area than subunits and better bonding inside multiple unit tablets.

However, tableting of multiple units together with pharmaceutically acceptable tableting excipients includes problem of particle segregation. Particle segregation in the tableting mixture results in tableting problems, such as weight variation and poor content uniformity. There are two approaches to solving this problem: tableting of multiple units together with direct compression excipients such as filler-binders, binders, disintegrants, lubricants, etc., and tableting of multiple units together with cushioning agglomerations obtained by agglomeration of particles of pharmaceutically acceptable excipients, such as fillers, binders, disintegrants, lubricants, etc.

In European Journal of pharmaceutics and Biopharmaceutics 47 (1999), 79-85 and 50 (2000), 285-291 is disclosed investigation of influence of five different microcrystalline filler-binders on the pellet distribution in multiple unit tablets, microcrystalline cellulose granules having mean particle size 1055 µm, 621 µm and 194 µm, powdered cellulose granules having mean particle size 179 µm and microcrystalline cellulose Avicel® PH 101, having mean particle size 54 µm. It was found that Avicel® PH 101 with a large surface area and a fibrous structure assures the best homogeneity of pellets distribution in multiple unit tablets.

In Pharmaceutical industry 58 (1996), 83-86 was also shown that the mixtures of pellets havin particle size range 800-1250 µm, with MCC grade Avicel® PH 101 having particle size about 50 µm, have less tendency to segregate at tableting than mixtures of the same pellets with Cellactose having particle size about 200 µm. This was explained by the shape and size of particles used.

In Powder Technology 96 (1998), 248-254 is disclosed preparation for multiple unit tablets comprising tableting of enteric coated pellets with microcrystalline cellulose granules and dicalcium phosphate granules, and with powders as filler-binders of different particle size. The mean diameter of the pellets was 1050 µm. Pellets were compacted together with one of direct compression excipients at the time, having mean particle size 50 µm, 100 µm and 190 µm, and with granules having particle size range 500-1000 µm. Pellets were compacted also with granules having particle size range 500-1000 µm with the addition of powder filler-binder having mean particle size 100 µm - Avicel® PH 102, but there was no significant difference between the mass and content uniformity of tablets with and without Avicel PH 102.

In WO 01/34684 A1 is disclosed preparation of multiple unit tablets comprising tableting of modified release multiple units together with porous microcrystalline cellulose cushioning granules obtained with wet granulation process of MCC with granulating fluid which contains a mixture of water and a volatile, water miscible, polar organic solvent. Cushioning MCC granules preferably have particle size similar to the particle size range of modified release multiple units.

In US 4 874 614 is disclosed use of microcrystalline cellulose as an excipient for tableting of friable coated drug granules.

In International Journal of Pharmaceutics 233 (2002), 67-83 is disclosed preparation of inert cushioning beads that provide a high degree of protection to modified release multiple units during compacting into multiple unit tablets. Inert cushioning beads are prepared by extrusion-spheronization followed by freeze drying in size ranges that provide minimal segregation propensity.

In European Journal of Pharmaceutical Sciences 17 (2002), 145-151 is disclosed preparation of cushioning wax beads that can minimize the damage to film-coated diltiazem hydrochloride pellets during compression step of tableting. Cushioning wax beads are based on paraffinic wax and starch derivatives and can be produced by melt pelletization and spray congealing.

In International Journal of Pharmaceutics 147 (1997), 95-107 is disclosed preparation of multiple unit tablets comprising compaction of three types of pellets, (a) those containing a model drug readily identifiable by colour, to evaluate tablet consistency; (b) those containing a deformable material, glyceryl monostearate, to provide pressure absorbing and binding properties, and (c) those containing an inorganic disintegrating agent.

From all said is concluded that there is need for developing coherent multiple unit tablets having appropriate friability and hardness, where drug release from multiple unit tablets is not altered compared to the drug release from multiple units prior to tableting and that the content and mass uniformity of multiple unit tablets complies with pharmacopoeia prescriptions.

The first embodiment of the present invention is a multiple unit tablet comprising:
(a) multiple units comprising at least one pharmaceutically active ingredient and
(b) a mixture of at least two tablet filler-binders and optionally other pharmaceutically acceptable excipients, wherein the particle size of the at least two filler-binders differs from each other, at least one of said tablet filler-binder being a bigger tablet filler-binder having mean particle size-to-mean multiple unit size ratio from 10% to 40%, and at least one of said tablet filler-binder being a smaller tablet filler-binder having mean particle size-to-mean multiple unit size ratio from 1% to 10%,

We have surprisingly found that the multiple unit tablets according to the present invention have appropriate hardness and friability, the drug release of the multiple unit tablets according to the present invention in not significantly altered compared to the drug release from multiple units prior to compaction and during the tableting of the multiple unit tablets according to the present invention segregation does not occur. The tableting mixture containing multiple units, tablet filler-binders and other pharmaceutically acceptable excipients remains homogeneous throughout the tableting of one production batch, which assures that the multiple unit tablets have good content and mass uniformity. Also the distribution of multiple units, tablet filler-binders and other pharmaceutically acceptable excipients, within individual tablets is uniform, which minimizes contacts of the multiple units with each other and results in every unit being surrounded with tablet filler-binder particles that absorb the compaction forces and in such ways minimizes the damage of multiple units caused by compression forces.

Multiple unit tablets according to the present invention comprise multiple units compacted together with at least two tablet filler-binders and optionally other pharmaceutically acceptable excipients, wherein the particle size of tablet filler-binders differs from each other and is smaller than the size of multiple units, preferably at least twice smaller than the size of multiple units. The size of multiple units-to-particle size of filler-binders ratio at the multiple unit tablets according to the present invention enables such spatial arrangement of multiple units, tablet filler-binders and other pharmaceutically acceptable excipients in the tableting mixture, which prevents particle segregation and assures homogeneous distribution of multiple units, tablet filler-binders and other pharmaceutically acceptable excipients in the tableting mixture within a production batch and in individual tablets.

The multiple units contained in the multiple unit tablets according to the present invention may be selected from the group consisting of pellets, granules, crystals, spherules, micro spheres, mini tablets and other agglomerations containing pharmaceutically active ingredient, preferably pellets, more preferably modified release pellets. The modified drug release may be sustained or delayed release. Sustained release may be achieved by matrix or by film coating, preferably by film coating. Delayed release is achieved by enteric coating. The multiple units' size may be 100-2000 µm, preferably 200-1200 µm, more preferably 500-1000 µm, most preferably about 750 µm.

Preferably, the multiple unit tablet according to the present invention comprises 30%-70% w/w, preferably 40%-60% w/w, more preferably 40%-50% w/w of multiple units.

The multiple unit tablets according to the present invention comprises at least two tablet filler-binders, preferably 2-5 tablet filler-binders, more preferably 2 or 3 tablet filler-binders.

A "tablet filler-binder" in the present invention means direct compression tablet diluent, that is usually common tablet diluent or a combination thereof, that is physically modified in order to be useful for direct compression tableting.

The multiple unit tablets according to the present invention comprises at least two tablet filler-binders having different particle size, wherein at least one of them is a bigger particle size tablet filler-binder and at least one of them is a smaller particle size tablet filler-binder.

A "bigger particle size tablet filler-binder" in the present invention means tablet filler-binder having mean particle size-to-mean multiple unit size ratio from 10% to 40%, preferably 15% to 30%, more preferably from 15% to 20%.

A "smaller particle size tablet filler-binder" in the present invention means tablet filler-binder having mean particle size-to-mean multiple unit size ratio from 1% to 10%, preferably from 2% to 10%, more preferably from 4% to 8%.

The amount of a bigger particle size tablet filler-binders in proportion to the total amount of a mixture of tablet filler-binders and other pharmaceutically acceptable excipients in the multiple unit tablets according to the present invention is about 50%-90% w/w, preferably about 50%-80% w/w, more preferably about 55%-80% w/w, most preferably about 60%-70% w/w.

The amount of smaller particle size tablet filler-binders in proportion to the total amount of a mixture of tablet filler-binders and other pharmaceutically acceptable excipients in the multiple unit tablets according to the present invention, is about 10%-50% w/w, preferably about 10%-45% w/w, more preferably about 20%-45% w/w, most preferably about 20%-35% w/w.

The tablet filler-binder in the multiple unit tablets according to the present invention may be tablet filler-binders selected from the group consisting of microcrystalline cellulose, powdered cellulose, lactose, sucrose, starch, polyalcohols, inorganic calcium salts, such as dicalcium phosphate dihydrate, anhydrous calcium phosphate, tricalcium phosphate, calcium sulphate dehydrate, calcium sulphate dihydrate, organic calcium salts such as calcium lactate, calcium lactate trihydrate and calcium lactate pentahydrate, polyethylene oxide or any commercially available combination thereof, preferably directly compressible lactose, microcrystalline cellulose and powdered cellulose, more preferably spray dried directly compressible lactose and microcrystalline cellulose.

Furthermore, the tablet filler-binder in the multiple unit tablets according to the present invention may be also prepared from microcrystalline cellulose, powdered cellulose, lactose, sucrose, starch, polyalcohols, inorganic calcium salts, such as dicalcium phosphate dihydrate, anhydrous calcium phosphate, tricalcium phosphate, calcium sulphate dehydrate, organic calcium salts such as calcium lactate, calcium lactate trihydrate and calcium lactate pentahydrate, polyethylene oxide or any combination thereof, by wet granulation, dry granulation, spray drying, spray congealing, freeze drying or other process used for obtaining particles or particle agglomerations of said excipients.

Preferably, a bigger particle size tablet filler-binder has globular particles and is preferably spray dried directly compressible lactose, such as Pharmatose^{®} DCL 14 or spray dried material composed of lactose and pulverized cellulose, such as Cellactose^{®} 80 or spray dried material composed of lactose and microcrystalline cellulose, such as MicroceLac^{®},

Preferably, the smaller particle size tablet filler-binder has fibrous particles and is a cellulose derivative, preferably microcrystalline cellulose and powdered cellulose, more preferably microcrystalline cellulose, such as Avicel^{®} PH 101 or Avicel^{®} PH 105.

The multiple unit tablets according to the present invention may comprise also other pharmaceutically acceptable excipients selected from the group consisting of disintegrants, lubricants, glidants and other pharmaceutically acceptable excipients commonly used in tableting. The amount of other pharmaceutically acceptable excipients in proportion to the total amount of a mixture of tablet filler-binders and other pharmaceutically acceptable excipients in the multiple unit tablets according to the present invention, is up to 10% w/w, preferably about 2%-10% w/w.

As disintegrants in the multiple unit tablets according to the present invention may be used croscarmellose sodium, carmellose sodium, carmellose calcicum, crospovidone, sodium starch glycolate, low-substituted hydroxypropyl cellulose, hydroxypropyl starch, etc., preferably crosscarmelose sodium.

As lubricants in the multiple unit tablets according to the present invention may be used, magnesium stearate, stearic acid, sodium stearyl fumarate, calcium stearate, zinc stearate, etc., preferably magnesium stearate or sodium stearyl fumarate.

Preferably, a pharmaceutical composition of the present invention comprises:
(a) 50%-90% w/w of tablet filler-binders having mean particle size-to-mean multiple unit size ratio from 10% to 40%, preferably 50%-50%
(b) 10%-50% w/w of tablet filler-binders having mean particle size-to-mean multiple unit size ratio from 1 % to 10%,
(c) optionally up to 10% of other pharmaceutically acceptable excipients, preferably 2%-10%.

The pharmaceutically active ingredient comprised in multiple units in multiple unit tablets according to the present invention may be selected from the group consisting of analgesics, anticonvulsants, antiparkinsonics, anaesthetics, antibiotics, antihypertensives, antihistaminics, antimalarial agents, antimigraine agents, anti-obesity agents, serum lipid reducing agents, antipyretics, alpha-blockers, alpha-adrenergic agonists, bactericides, bronchial dilators, beta-adrenergic stimulants, beta-adrenergic blockers, enzymes, contraceptives, cardiovascular active substances, calcium channel inhibitors, proton pump inhibitors, diuretics, hypnotics, hormones, hyperglycemics, hypoglycemics, muscle relaxants and contractors, parasympathomimetics, sedatives, sympathomimetics, tranquillizers, vitamins and any combinations thereof. Preferably, the pharmaceutically active ingredient is proton pump inhibitor, such as omeprazole, esomeprazole, lansoprazole, rabeprazole, pantoprazole or their pharmaceutically acceptable salts such as sodium and magnesium salts. More preferably, the pharmaceutically active ingredient is esomeprazole or its salts.

Preferred multiple unit tablet according to the present invention comprises:
(a) 30%-70% w/w of multiple units, preferably pellets, more preferably modified release pellets,
(b) 30%-70% w/w of a mixture of tablet filler-binders and optionally other pharmaceutically acceptable excipients comprising:
   (b1) 50%-90% w/w of at least one bigger particle size filler-binder, preferably having globular particles,
   (b2) 10%-50% w/w of at least one smaller particle size filler-binders, preferably having fibrous particles, more preferably microcrystalline cellulose,
   (b3) optionally up to 10% w/w of other pharmaceutically acceptable excipients.

In another option, preferred multiple unit tablet according to the present invention comprises:
(a) 30%-70% w/w of multiple units, preferably pellets, more preferably modified release pellets,
(b) 30%-70% w/w of a mixture of tablet filler-binders and other pharmaceutically acceptable excipients comprising:
   (b1) 50%-80% w/w of at least one bigger particle size filler-binder, preferably having globular particles,
   (b2) 10%-50% w/w of at least one smaller particle size filler-binders, preferably having fibrous particles, more preferably microcrystalline cellulose,
   (b3) 2%-10% w/w of other pharmaceutically acceptable excipients.

More preferred multiple unit tablet according to the present invention comprises:
(a) 40%-50% w/w of modified release pellets having mean particle size about 500-1000 µm,
(b) 50%-60% w/w of a mixture of tablet filler-binders and other pharmaceutically acceptable excipients comprising:
   (b1) 55%-80% w/w of bigger particle size filler-binder with globular particles having mean particle size about 100-200 µm, preferably spray dried directly compressible lactose or spray dried material composed of lactose and pulverized cellulose, or spray dried material composed of lactose and microcrystalline cellulose,
   (b2) 15%-25% w/w of first smaller particle size filler-binder with fibrous particles having mean particle size about 50 µm, preferably microcrystalline cellulose,
   (b3) 5%-20% w/w of second smaller particle size filler-binder with fibrous particles having mean particle size about 20 µm, preferably microcrystalline cellulose,
   (b4) optionally up to 10% w/w of other pharmaceutically acceptable excipients, as for example about 2.0-9.5% w/w of disintegrant and about 0.5% w/w of lubricant.

In another option, more preferred multiple unit tablet according to the present invention comprises:
(a) 40%-50% w/w of modified release pellets having mean particle size about 500-1000 µm,
(b) 50%-60% w/w of a mixture of tablet filler-binders and other pharmaceutically acceptable excipients comprising:
   (b1) 60%-70% w/w of bigger particle size filler-binder with globular particles having mean particle size about 100-200 µm, preferably spray dried directly compressible lactose or spray dried material composed of lactose and pulverized cellulose, or spray dried material composed of lactose and microcrystalline cellulose,
   (b2) 15%-25% w/w of first smaller particle size filler-binder with fibrous particles having mean particle size about 50 µm, preferably microcrystalline cellulose,
   (b3) 5%-15% w/w of second smaller particle size filler-binder with fibrous particles having mean particle size about 20 µm, preferably microcrystalline cellulose,
   (b4) 5%-10% w/w of other pharmaceutically acceptable excipients, preferably about 4.5-9.5% w/w of disintegrant and about 0.5% w/w of lubricant..

In another option, more preferred multiple unit tablet according to the present invention comprises:
(a) 40%-50% w/w of modified release pellets having mean particle size about 500-1000 µm,
(b) 50%-60% w/w of a mixture of tablet filler-binders and other pharmaceutically acceptable excipients comprising:
   (b1) 55%-80% w/w of bigger particle size filler-binder with globular particles having mean particle size about 100-200 µm, preferably spray dried directly compressible lactose or spray dried material composed of lactose and pulverized cellulose, or spray dried material composed of lactose and microcrystalline cellulose,
   (b2) 20%-45% w/w of smaller particle size filler-binder with fibrous particles having mean particle size about 20 µm, preferably microcrystalline cellulose,
   (b3) optionally up to 10% w/w of other pharmaceutically acceptable excipients, preferably about 2.0-9.5% w/w of disintegrant and about 0.5% w/w of lubricant.

In another option, more preferred multiple unit tablet according to the present invention comprises:
(a) 40%-50% w/w of modified release pellets having mean particle size about 500-1000 µm,
(b) 50%-60% w/w of a mixture of tablet filler-binders and other pharmaceutically acceptable excipients comprising:
   (b1) 60%-70% w/w of bigger particle size filler-binder with globular particles having mean particle size about 100-200 µm, preferably spray dried directly compressible lactose or spray dried material composed of lactose and pulverized cellulose, or spray dried material composed of lactose and microcrystalline cellulose,
   (b2) 20%-35% w/w of smaller particle size filler-binder with fibrous particles having mean particle size about 20 µm, preferably microcrystalline cellulose,
   (b3) 5%-10% w/w of other pharmaceutically acceptable excipients, preferably about 4.5-9.5% w/w of disintegrant and about 0.5% w/w of lubricant.

Second embodiment of the present invention is related to a process for the preparation of multiple unit tablets of the present invention comprising the following steps of:
(a) mixing together said multiple units and a mixture of at least two tablet filler-binders and optionally other pharmaceutically acceptable excipients,
(b) compacting tableting mixture obtained from step (a).

The multiple unit tablets according to the present invention are prepared by mixing of multiple units, filler-binders, and optionally disintegrant, lubricant and other pharmaceutically acceptable excipients and compacting such obtained tableting mixture by rotary tabletting machine. The mixing of tableting mixture components is carried out preferably in double cone mixer. The compaction of multiple unit tablets according to the present invention is carried out by rotary tabletting machine and preferably includes both precompresion and main compression step.

The following examples illustrate the invention, but do not limit it in any way:

### Example 1

### Preparation of enteric coated esomeprazole pellets

**CORE MATERIAL (weight 233,376 mg)**

| | | |
|---|---|---|
| Non-pareil beads | 124.576 mg | |
| Esomeprazole magnesium | 40.000 mg | Active substance |
| Polyvinylpyrrolidone K-25 | 64.000 mg | Polymer carrier |
| Dibutyl sebacate | 4.480 mg | Plasticizer |
| Polysorbate | 0.320 mg | Surface active agent |
| Ethanol | 875.816 mg | Organic solvent |

### Method of preparation of a core material

Polysorbate, polyvinylpyrrolidone K-25, dibutyl sebacate, and esomeprazole magnesium are dissolved in ethanol. The obtained solution is bottom sprayed onto non-pareil beads in fluid-bed device. During this process ethanol evaporates and solid solution of esomeprazole magnesium in polyvinylpyrrolidone is formed as a layer on the surface of non-pareil beads.
The obtained core material is dried in fluid-bed device.

**SUBCOATING (12% application, coating weight 31.824 mg)**

| | | |
|---|---|---|
| Polyvinylpyrrolidone K-25 | 7.956 mg | Film forming polymer |
| Ethyl cellulose | 1.592 mg | Film forming polymer |
| Dibutyl sebacate | 0.954 mg | Plasticizer |
| Talcum | 21.322 mg | Anti-tacking agent |
| Ethanol | 233.376 mg | Organic solvent |

### Method of preparation of subcoating

Polyvinylpyrrolidone, ethyl cellulose and dibutyl sebacate are dissolved in ethanol. Talcum is suspended in the obtained solution. The obtained dispersion is bottom sprayed in fluid-bed device onto the core material.

**ENTERIC COATING (25% application, coating weight 88.400 mg)**

| | | |
|---|---|---|
| Hydroxypropyl methylcellulose phthalate HP-55 | 53.040 mg | Enteric polymer |
| Dibutyl sebacate | 5.304 mg | Plasticizer |
| Talcum | 30.056 mg | Anti-tacking agent |
| Ethanol | 635.800 mg | Organic solvent |
| Acetone | 635.800 mg | Organic solvent |

### Method of preparation of enteric coating

Hydroxypropyl methylcellulose phthalate HP-55 and dibutyl sebacate are dissolved in the mixture of ethanol and acetone. Talcum is suspended in obtained solution. The dispersion is bottom sprayed in fluid-bed device onto the core material covered with subcoating.

### Example 2

### Preparation of enteric coated esomeprazole pellets

**CORE MATERIAL (weight 251,160 mg)**

| | | |
|---|---|---|
| Non-pareil beads | 139.800 mg | |
| Esomeprazole magnesium | 40.000 mg | Active substance |
| Polyvinylpyrrolidone K-25 | 64.000 mg | Polymer carrier |
| Dibutyl sebacate | 4.608 mg | Plasticizer |
| Polysorbate | 2.752 mg | Surface active agent |
| Ethanol | 802.926 mg | Organic solvent |

### Method of preparation of a core material

Polysorbate, polyvinylpyrrolidone K-25, dibutyl sebacate, and esomeprazole magnesium are dissolved in ethanol. The obtained solution is bottom sprayed onto non-pareil beads in fluid-bed device. During this process ethanol evaporates and solid solution of esomeprazole magnesium in polyvinylpyrrolidone is formed as a layer on the surface of non-pareil beads.
The obtained core material is dried in fluid-bed device.

**SUBCOATING (8% application, coating weight 21.840 mg)**

| | | |
|---|---|---|
| Polyvinylpyrrolidone K-25 | 7.382 mg | Film forming polymer |
| Dibutyl sebacate | 0.874 mg | Plasticizer |
| Talcum | 13.584 mg | Anti-tacking agent |
| Ethanol | 196.560 mg | Organic solvent |

### Method of preparation of subcoating

Polyvinylpyrrolidone and dibutyl sebacate are dissolved in ethanol. Talcum is suspended in the obtained solution. The obtained dispersion is bottom sprayed in fluid-bed device onto the core material.

**ENTERIC COATING (25% application, coating weight 91.000 mg)**

| | | |
|---|---|---|
| Hydroxypropyl methylcellulose phthalate HP-55 | 54.600 mg | Enteric polymer |
| Dibutyl sebacate | 4.550 mg | Plasticizer |
| Talcum | 27.300 mg | Anti-tacking agent |
| Glyceryl monostearate | 4.550 mg | Anti-tacking agent |
| Ethanol | 664.286 mg | Organic solvent |
| Acetone | 664.286 mg | Organic solvent |

### Method of preparation of enteric coating

Hydroxypropyl methylcellulose phthalate HP-55 and dibutyl sebacate are dissolved in the mixture of ethanol and acetone. Talcum and glyceryl monostearate are suspended in obtained solution. The dispersion is bottom sprayed in fluid-bed device onto the core material covered with subcoating.

### Example 3

### Preparation of multiple unit tablets containing enteric coated esomeprazole pellets

| | Weight per tablet (mg) | Mean particle size (µm) | Portion in formulation |
|---|---|---|---|
| Enteric coated pellets according to example 1 | 353.000 | 750 | 44.2 % (of tablet) |
| Pharmatose DCL 14 | 268.000 | 125 | 60.0% (of tableting excipients) |
| Avicel PH 105 | 151.776 | 20 | 34.0% (of tableting excipients) |
| AcDiSol | 24.585 | | 5.5% (of tableting excipients) |
| Mg stearate | 2.232 | | 0.5% (of tableting excipients) |
| | | | |
| Tableting excipients* | 446.400 | | |
| Tablet weight | 800.000 | | |

| | | | |
|---|---|---|---|
| * The total amount of tablet filler-binders and other pharmaceutically acceptable excipients. | | | |

### Method of preparation of tablets

Enteric coated pellets according to example 1, Pharmatose DCL 14, Avicel PH 105 and AcDiSol are mixed for 15 minutes in double cone mixer. Mg stearate is added and mixing is continued for 1 more minute. Tableting mixture is compacted by rotary tablet machine, using both precompression and main compression step.

### Example 4

### Preparation of multiple unit tablets containing enteric coated esomeprazole pellets

| | Weight per tablet (mg) | Mean particle size (µm) | Portion in formulation |
|---|---|---|---|
| Enteric coated pellets according to example 1 | 353.600 | 750 | 44.2% (of tablet) |
| Pharmatose DCL 14 | 290.160 | 125 | 65% (of tableting excipients) |
| Avicel PH 101 | 83.700 | 50 | 18.8% (of tableting excipients) |
| Avicel PH 105 | 27.900 | 20 | 6.3% (of tableting excipients) |
| AcDiSol | 42.408 | | 9.5% (of tableting excipients) |
| Mg stearat | 2.232 | | 0.5% (of tableting excipients) |
| | | | |
| Tableting excipients* | 446.400 | | |
| Tablet weight | 800.000 | | |

| | | | |
|---|---|---|---|
| * The total amount of tablet filler-binders and other pharmaceutically acceptable excipients. | | | |

### Method of preparation of tablets

Enteric coated pellets according to example 1, Pharmatose DCL 14, Avicel PH 101, Avicel PH 105 and AcDiSol are mixed for 15 minutes in double cone mixer. Mg stearate is added and mixing is continued for 1 more minute. Tableting mixture is compacted by rotary tablet machine, using both precompression and main compression step.

### Example 5

### Preparation of multiple unit tablets containing enteric coated esomeprazole pellets

| | Weight per tablet (mg) | Mean particle size (µm) | Portion in formulation |
|---|---|---|---|
| Enteric coated pellets according to example 1 | 353.600 | 750 | 44.2% (of tablet) |
| Pharmatose DCL 14 | 267.840 | 125 | 60% (of tableting excipients) |
| Avicel PH 101 | 98.208 | 50 | 22% (of tableting excipients) |
| Avicel PH 105 | 53.568 | 20 | 12% (of tableting excipients) |
| AcDiSol | 24.552 | | 5.5% (of tableting excipients) |
| Mg stearat | 2.232 | | 0.5% (of tableting excipients) |
| Tableting excipients* | 446.400 | | |
| Tablet weight | 800.000 | | |

| | | | |
|---|---|---|---|
| * The total amount of tablet filler-binders and other pharmaceutically acceptable excipients. | | | |

### Method of preparation of tablets

Enteric coated pellets according to example 1, Pharmatose DCL 14, Avicel PH 101, Avicel PH 105 and AcDiSol are mixed for 15 minutes in double cone mixer. Mg stearate is added and mixing is continued for 1 more minute. Tableting mixture is compacted by rotary tablet

### Comparative example 1

| | Weight per tablet (mg) | Mean particle size (µm) | Portion in formulation |
|---|---|---|---|
| Enteric coated pellets according to example 2 | 364.000 | 750 | 47.3% (of tablet) |
| Pharmatose DCL 22 | 160.000 | 150 | 39.4% (of tableting excipients) |
| Avicel PH 102 | 220.000 | 100 | 54.2% (of tableting excipients) |
| AcDiSol | 22.000 | | 5.4% (of tableting excipients) |
| Mg stearate | 4.000 | | 1.0% (of tableting excipients) |
| | | | |
| Tableting excipients* | 406.000 | | |
| Tablet weight | 770.000 | | |

| | | | |
|---|---|---|---|
| * The total amount of tablet filler-binders and other pharmaceutically acceptable excipients. | | | |

### Method of preparation of tablets

Enteric coated pellets according to example 2, Pharmatose DCL 22, Avicel PH 102, and AcDiSol are mixed for 15 minutes in double cone mixer. Mg stearate is added and mixing is continued for 1 more minute. Tableting mixture is compacted by rotary tablet machine, using both precompression and main compression step.

### Comparative example 2

| | Weight per tablet (mg) | Mean particle size (µm) | Portion in formulation |
|---|---|---|---|
| Enteric coated pellets according to example 2 | 364.000 | 750 | 47.3% (of tablet) |
| Avicel PH 200 | 392.000 | 190 | 96.5% (of tableting excipients) |
| AcDiSol | 10.000 | | 2.5% (of tableting excipients) |
| Mg stearate | 4.000 | | 1% (of tableting excipients) |
| | | | |
| Tableting excipients* | 406.000 | | |
| Tablet weight | 770.000 | | |

| | | | |
|---|---|---|---|
| * The total amount of tablet filler-binders and other pharmaceutically acceptable excipients. | | | |

### Method of preparation of tablets

Enteric coated pellets according to example 2, Avicel PH 200, and AcDiSol are mixed for 15 minutes in double cone mixer. Mg stearate is added and mixing is continued for 1 more minute. Tableting mixture is compacted by rotary tablet machine, using both precompression and main compression step.

### Multiple unit tablets properties

**Table 1. Comparison of friability of multiple unit tablets according to present invention with multiple unit tablets according to comparative examples 1 and 2**

| Multiple unit tablets compsition | Friability |
|---|---|
| Example 3 | 0.8% |
| Example 4 | 1 % |
| Example 5 | 0.6% |
| Comparative example 1 | 4.1% |
| Comparative example 2 | 3.5% |

The friability of multiple unit tablets according to present invention is better than friability of multiple unit tablets according to comparative examples 1 and 2. This shows that pellets in multiple unit tablets according to present invention are cohesive and can withstand further processing, such as coating and packing.

**Table 2. Comparison of content of active pharmaceutical substance and mass at the beginning and at the end of tableting of 1.6 kg batch of multiple unit tablets according to present invention with content of active pharmaceutical substance and mass at the beginning and at the end of tableting of 1.6 kg batch of the multiple unit tablets according to comparative examples 1 and 2**

| Multiple unit tablets composition | The beginning of tableting | | The end of tableting | |
|---|---|---|---|---|
| | Content of active substance (mg) | Mass (mg) | Content of active substance (mg) | Mass (mg) |
| Example 3 | 41.52 | 818.3 | 39.84 | 802.6 |
| Example 4 | 39.58 | 786.2 | 41.12 | 810.5 |
| Example 5 | 41.13 | 809.7 | 39.52 | 794.5 |
| Comparative example 1 | 46.79 | 804.6 | 40.02 | 743.3 |
| Comparative example 2 | 44.27 | n.a. | 39.75 | n.a. |

The results presented in the table 2 show that the active substance content and tablet mass is not significantly changed during the tableting of one laboratory batch (batch size 1.6 kg) of multiple unit tablets according to the present invention, while the active substance content and tablet mass is significantly changed during the tableting of multiple unit tablets according to comparative examples 1 and 2. These results show that significant particle segregation does not occur in the tableting mixture containing multiple units, tablet filler-binders and other pharmaceutically acceptable excipients according to the present invention, so said tableting mixture remains homogeneous throughout the tableting of one laboratory batch (batch size 1.6 kg), which assures that the multiple unit tablets have good content and mass uniformity.

**Table 3. Comparison of the acid resistance of the multiple unit tablets according to present invention with the acid resistance of the multiple unit tablets according to comparative examples 1 and 2**

| Multiple unit tablets compsition | Acid resistance (% dissolved in 0,1 M HCl) |
|---|---|
| Example 3 | 3.4 |
| Example 4 | 6.1 |
| Example 5 | 4.3 |
| Comparative example 1 | 23.5 |
| Comparative example 2 | 19.8 |

Results presented in table 3 show that the acid resistance of multiple unit tablets according to present invention complies with pharmacopoeia requirements, while the acid resistance of the multiple unit tablets according to comparative examples 1 and 2 does not. These results show that the distribution of multiple units, tablet filler-binders and other pharmaceutically acceptable excipients, within individual tablets according to present invention is uniform, which minimizes contacts of the multiple units with each other and results in every unit being surrounded with tablet compressible particles of filler-binders, that absorb the compaction forces and in such way minimizes the damage of multiple units caused by compression forces.

## Claims

1. A multiple unit tablet comprising:
(a) multiple units comprising at least one pharmaceutically active ingredient and
(b) a mixture of at least two tablet filler-binders and optionally other pharmaceutically acceptable excipients, wherein the particle size of the at least two filler-binders differs from each other, at least one of said tablet filler-binder being a bigger tablet filler-binder having mean particle size-to-mean multiple unit size ratio from 10% to 40%, and at least one of said tablet filler-binder being a smaller tablet filler-binder having mean particle size-to-mean multiple unit size ratio from 1% to 10%,
and wherein the amount of bigger tablet filler-binder is 50%-90% w/w and the amount of smaller tablet filler-binder is 10%-50%, in proportion to the total amount of a mixture of tablet filler-binders and other pharmaceutically acceptable excipients in the multiple unit tablet.

2. A multiple unit tablet according to claim 1, wherein said mixture of at least two tablet filler-binders comprises up to 10% of other pharmaceutically acceptable excipients.

3. A multiple unit tablet according to any one of claims 1-2, comprising:
(a) 30%-70% w/w of multiple units,
(b) 30%-70% w/w of a mixture of tablet filler-binders and other pharmaceutically acceptable excipients comprising:
(b1) 50%-90% w/w of at least one tablet filler-binder with globular particles having mean particle size-to-multiple unit size ratio from 10% to 40%,
(b2) 10%-50% w/w of at least one tablet filler-binder with fibrous particles having mean particle size-to-mean multiple unit size ratio from 1% to 10%,
(b3) optionally up to 10% w/w of other pharmaceutically acceptable excipients.

4. A multiple unit tablet according to any one of claims 1-3, comprising:
(a) 40%-50% w/w of modified release pellets having mean particle size 500-1000 µm,
(b) 50%-60% w/w of a mixture of tablet filler-binders and other pharmaceutically acceptable excipients, comprising:
(b1) 55%-80% w/w of tablet filler-binder with globular particles having mean particle size 100-200 µm,
(b2) 15%-25% w/w of tablet filler-binder with fibrous particles having mean particle size 50 µm,
(b3) 5%-20% w/w of tablet filler-binder with fibrous particles having mean particle size 20 µm,
(b4) optionally up to 10% w/w of other pharmaceutically acceptable excipients.

5. A multiple unit tablet according to any one of claims 1-3, comprising
(a) 40%-50% w/w of modified release pellets having mean particle size 500-1000 µm,
(b) 50%-60% w/w of a mixture of tablet filler-binders and other pharmaceutically acceptable excipients, comprising:
(b1) 55%-80% w/w of tablet filler-binder with globular particles having mean particle size 100-200 µm,
(b2) 20%-45% w/w of smaller particle size filler-binder with fibrous particles having mean particle size 20 µm,
(b3) optionally up to 10% w/w of other pharmaceutically acceptable excipients.

6. A multiple unit tablet according claim 2, comprising:
(a) 50%-80% w/w of tablet filler-binders having mean particle size-to-mean multiple unit size ratio from 10% to 40%,
(b) 10%-50% w/w of tablet filler-binders having mean particle size-to-mean multiple unit size ratio from 2% to 10%,
(c) 2%-10% of other pharmaceutically acceptable excipients.

7. A multiple unit tablet according to any one of claims 3 to 5, wherein said tablet filler-binder with globular particles is selected from group consisting of spray dried directly compressible lactose, spray dried material composed of lactose and pulverized cellulose, and spray dried material composed of lactose and microcrystalline cellulose.

8. A multiple unit tablet according to any one of claims 3 to 5, wherein said tablet filler-binder with fibrous particles is microcrystalline cellulose.

9. A multiple unit tablet according to any one of claims 1 to 8, wherein said pharmaceutically active ingredient is a proton pump inhibitor.

10. A multiple unit tablet according to claim 8, wherein said proton pump inhibitor is esomeprazole or its salt.

11. A process for the preparation of a multiple unit tablet according to any one of claims 1 to 7, comprising steps of:
(a) mixing together said multiple units and a mixture of at least two tablet filler-binders and optionally other pharmaceutically acceptable excipients,
(b) compacting of tableting mixture obtained from step (a).

## Patentansprüche

1. Eine Tablette mit mehreren Einheiten (*multiple units;* Mehrfacheinheiten), umfassend:
(a) mehrere Einheiten, umfassend mindestens einen pharmazeutisch aktiven Inhaltsstoff, und
(b) eine Mischung von mindestens zwei Tabletten-Füllstoff-Bindemitteln und optional anderen pharmazeutisch akzeptablen Hilfsstoffen, wobei sich die Partikelgröße der mindestens zwei Füllstoff-Bindemittel voneinander unterscheidet, wobei mindestens eines dieser Tabletten-Füllstoff-Bindemittel ein größeres Tabletten-Füllstoff-Bindemittel mit einem Verhältnis der mittleren Partikelgröße-zu-mittlerer Größe der Mehrfacheinheiten von 10% bis 40% ist, und mindestens eines dieser Tabletten-Füllstoff-Bindemittel ein kleineres Tabletten-Füllstoff-Bindemittel mit einem Verhältnis der mittleren Partikelgröße-zu-mittlerer Größe der Mehrfacheinheiten von 1% bis 10% ist,
und wobei die Menge des größeren Tabletten-Füllstoff-Bindemittels 50%-90% Gew./Gew. beträgt und die Menge des kleineren Tabletten-Füllstoff-Bindemittels 10%-50% beträgt, im Verhältnis zu der Gesamtmenge einer Mischung aus Tabletten-Füllstoff-Bindemitteln und anderen pharmazeutisch akzeptablen Hilfsstoffen in der Tablette mit mehreren Einheiten.

2. Eine Tablette mit mehreren Einheiten gemäß Anspruch 1, wobei diese Mischung der mindestens zwei Tabletten-Füllstoff-Bindemittel bis zu 10% anderer pharmazeutisch akzeptabler Hilfsstoffe enthält.

3. Tablette mit mehreren Einheiten gemäß irgendeinem der Ansprüche 1-2, umfassend:
(a) 30%-70% Gew./Gew. mehrerer Einheiten,
(b) 30%-70% Gew./Gew. einer Mischung aus Tabletten-Füllstoff-Bindemitteln und anderen pharmazeutisch akzeptablen Hilfsstoffen, umfassend:
(b1) 50%-90% w/w mindestens eines Tabletten-Füllstoff-Bindemittels mit kugelförmigen Partikeln mit einem Verhältnis der mittleren Partikelgröße-zu-Größe der Mehrfacheinheiten von 10% bis 40%,
(b2) 10%-50% Gew./Gew. mindestens eines Tabletten-Füllstoff-Bindemittels mit faserförmigen Partikeln mit einem Verhältnis der mittleren Partikelgröße-zu-mittlerer Größe der Mehrfacheinheiten von 1% bis 10%,
(b3) optional bis zu 10% Gew./Gew. anderer pharmazeutisch akzeptabler Hilfsstoffe.

4. Tablette mit mehreren Einheiten gemäß irgendeinem der Ansprüche 1-3, umfassend:
(a) 40%-50% Gew./Gew. Pellets mit modifizierter Freisetzung mit einer mittleren Partikelgröße von 500-1000 µm,
(b) 50%-60% Gew./Gew. einer Mischung von Tabletten-Füllstoff-Bindemitteln und anderen pharmazeutisch akzeptablen Hilfsstoffen, umfassend:
(b1) 55%-80% Gew./Gew. Tabletten-Füllstoff-Bindemittel mit kugelförmigen Partikeln mit einer mittleren Partikelgröße von 100-200 µm,
(b2) 15%-25% Gew./Gew. Tabletten-Füllstoff-Bindemittel mit faserförmigen Partikeln mit einer mittleren Partikelgröße von 50 µm,
(b3) 5%-20% Gew./Gew. Tabletten-Füllstoff-Bindemittel mit faserförmigen Partikeln mit einer mittleren Partikelgröße von 20 µm,
(b4) optional bis zu 10% Gew./Gew. anderer pharmazeutisch akzeptabler Hilfsstoffe.

5. Tablette mit mehreren Einheiten gemäß irgendeinem der Ansprüche 1-3, umfassend:
(a) 40%-50% Gew./Gew. Pellets mit modifizierter Freisetzung mit einer mittleren Partikelgröße von 500-1000 µm,
(b) 50%-60% Gew./Gew. einer Mischung von Tabletten-Füllstoff-Bindemitteln und anderer pharmazeutisch akzeptabler Hilfsstoffe, umfassend:
(b1) 55%-80% Gew./Gew. Tabletten-Füllstoff-Bindemittel mit kugelförmigen Partikeln mit einer mittleren Partikelgröße von 100-200 µm,
(b2) 20%-45% Gew./Gew. Füllstoff-Bindemittel mit kleinerer Partikelgröße mit faserförmigen Partikeln mit einer mittleren Partikelgröße von 20 µm,
(b3) optional bis zu 10% Gew./Gew. anderer pharmazeutisch akzeptabler Hilfsstoffe.

6. Tablette mit mehreren Einheiten gemäß Anspruch 2, umfassend:
(a) 50%-80% Gew./Gew. Tabletten-Füllstoff-Bindemittel mit einem Verhältnis der mittleren Partikelgröße-zu-mittlerer Größe der Mehrfacheinheiten von 10% bis 40%,
(b) 10%-50% Gew./Gew. Tabletten-Füllstoff-Bindemittel mit einem Verhältnis der mittleren Partikelgröße-zu-mittlerer Größe der Mehrfacheinheiten von 2% to 10%,
(c) 2%-10% anderer pharmazeutisch akzeptabler Hilfsstoffe.

7. Tablette mit mehreren Einheiten gemäß irgendeinem der Ansprüche 3 bis 5, wobei dieses Tabletten-Füllstoff-Bindemittel mit kugelförmigen Partikeln ausgewählt ist aus der Gruppe bestehend aus sprühgetrockneter direkt komprimierbarer Laktose, sprühgetrocknetem Material zusammengesetzt aus Laktose und pulverisierter Cellulose, und sprühgetrocknetem Material zusammengesetzt aus Laktose und mikrokristalliner Cellulose.

8. Tablette mit mehreren Einheiten gemäß irgendeinem der Ansprüche 3 bis 5, wobei dieses Tabletten-Füllstoff-Bindemittel mit faserförmigen Partikeln mikrokristalline Cellulose ist.

9. Tablette mit mehreren Einheiten gemäß irgendeinem der Ansprüche 1 bis 8, wobei dieser pharmazeutisch aktive Inhaltsstoff ein Protonenpumpeninhibitor ist.

10. Tablette mit mehreren Einheiten gemäß Anspruch 8, wobei dieser Protonenpumpeninhibitor Esomeprazol oder sein Salz ist.

11. Verfahren zur Herstellung einer Tablette mit mehreren Einheiten gemäß irgendeinem der Ansprüche 1 bis 7, umfassend Schritte von:
(a) Zusammenmischen dieser mehreren Einheiten und einer Mischung von mindestens zwei Tabletten-Füllstoff-Bindemitteln und optional anderen pharmazeutisch akzeptablen Hilfsstoffen,
(b) Kompaktieren der aus Schritt (a) erhaltenen Tablettierungs-Mischung.

## Revendications

1. Un comprimé à unités multiples comprenant :
(a) Des unités multiples comprenant au moins un ingrédient pharmaceutique actif et
(b) Un mélange d'au moins deux charge-liants de comprimé et de façon optionnelle d'autres excipients acceptables d'un point de vue pharmaceutique , où la taille des particules d'au moins deux charge-liants diffère l'un de l'autre, au moins un desdits charge-liants de comprimé étant un charge-liant de comprimé plus important ayant un ratio de taille de particule par rapport à la taille moyenne des unité multiples entre 10 % et 40 % et au moins un charge-liant de comprimé étant un charge-liant de comprimé moins important ayant un ratio de taille de particule par rapport à taille moyenne des unités multiples entre 1% et 10 %,
Et où la quantité de charge-liant de comprimé plus important se situe entre 50% et 90% poids/poids et la quantité de charge-liant de comprimé moins important se situe entre 50% et 90% poids/poids se situe entre 10 % et 50% par rapport à la quantité totale d'un mélange de charge-liant de comprimé et d'autres excipients acceptables du point de vue pharmaceutique dans le comprimé à unités multiples.

2. Un comprimé à unités multiples selon la revendication 1, où ledit mélange d'au moins deux charge-liants de comprimé comprend jusqu'à 10% des autres excipients acceptables du point de vue pharmaceutique.

3. Un comprimé à unités multiples selon une quelconque des revendications 1-2, comprenant :
(a) 30% - 70 % poids/poids des unités multiples,
(b) 30% - 70 % poids/poids d'un mélange de charge-liant de comprimé et d'autres excipients acceptables du point de vue pharmaceutique, comprenant :
(b1) 50 % - 90 % poids/poids d'au moins un charge-liant de comprimé avec des particules sphériques ayant un ratio entre la taille moyenne de particules par rapport à la taille des unités multiples entre 10 % et 40 %.
(b2) 10 % - 50 % poids/poids d'au moins un charge-liant de comprimé avec des particules FIBROUS ayant un ratio entre la taille moyenne de particules par rapport à la taille moyenne des unités multiples entre 1 % et 10 %.
(b3) de façon optionnelle jusqu'à 10 % poids/poids d'autres excipients acceptables du point de vue pharmaceutique.

4. Un comprimé à unités multiples selon une quelconque des revendications 1-3, comprenant :
(a) 40 % - 50 % de billes modifiées à libération ayant une taille moyenne de particule entre 500 et 1000 µm,
(b) 50 % - 60 % poids/poids d'un mélange de charge-liants de comprimé et d'autres excipients acceptables de point de vue pharmaceutique, comprenant :
(b1) 55 % - 80 % d'un charge-liant de comprimé avec des particules sphériques ayant une taille de particules moyenne entre 100 et 200 µm,
(b2) 15 % - 25 % d'un charge-liant de comprimé avec des particules fibreuses ayant une taille de particules moyenne de 50 µm,
(b3) 5 % - 20 % d'un charge-liant de comprimé avec des particules fibreuses ayant une taille de particules moyenne de 20 µm,
(b4) de façon optionnelle jusqu'à 10 % poids/poids d'autres excipients acceptables du point de vue pharmaceutique.

5. Un comprimé à unités multiples selon une quelconque des revendications 1-3, comprenant :
(a) 40 % - 50 % de billes modifiés à libération ayant une taille moyenne de particule entre 500 et 1000 µm,
(b) 50 % - 60 % poids/poids d'un mélange de charge-liants de comprimé et d'autres excipients acceptables de point de vue pharmaceutique, comprenant :
(b1) 55 % - 80 % de charge-liant de comprimé avec des particules sphériques ayant une taille de particules moyenne entre 100 et 200 µm,
(b2) 20 % - 45 % poids/poids de charge-liant d'une taille de particules moins important avec des particules fibreuses ayant une taille de particules moyenne de 20 µm,
(b3) de façon optionnelle jusqu'à 10 % poids/poids d'autres excipients acceptables du point de vue pharmaceutique

6. Un comprimé à unités multiples selon la revendication 2, comprenant :
(a) 50 % - 80 % poids/poids de charge-liant de comprimé ayant un ratio de la taille moyenne des particules par rapport à la taille moyenne des unités multiples entre 10 % et 40 %,
(b) 10 % - 50 % poids/poids de charge-liant de comprimé ayant un ratio de la taille moyenne des particules par rapport à la taille moyenne des unités multiples entre 2 % et 10 %,
(c) 2 % - 10 % d'autres excipients acceptables du point de vue pharmaceutique.

7. Un comprimé à unités multiples selon une quelconque des revendications 3 - 5, où ledit charge-liant de comprimé ayant des particules sphériques est sélectionné parmi le groupe comportant de lactose séchée par pulvérisation et étant directement compressible, un matériau étant séché par pulvérisation et étant composé de lactose et d'une cellulose pulvérisée et un matériau séché par pulvérisation et composé de lactose et de cellulose microcristalline.

8. Un comprimé à unités multiples selon une quelconque des revendications 3 - 5, où ledit charge-liant de comprimé ayant des particules fibreuses est de la cellulose microcristalline.

9. Un comprimé à unités multiples selon une quelconque des revendications 1 - 8, où ledit ingrédient actif du point de vue pharmaceutique est un inhibiteur de pompe à protons.

10. Un comprimé à unités multiples selon la revendication 8, où ledit inhibiteur de pompe à protons est de l'ézoméprazole ou un de ses sels.

11. Un procédé destiné à fabriquer un comprimé à unités multiples selon une quelconque des revendications 1- 7, comprenant les étapes suivantes :
(a) mélange desdites unités multiples avec un mélange d'au moins deux charge-liants de comprimé et de façon optionnelle d'autres excipients acceptable du point de vue pharmaceutique,
(b) compactage du mélange destiné à fabriquer les comprimés obtenu à partir de l'étape (a).
